# FASCICULE DE BREVET EUROPEEN

(11) **EP 0 560 969 B1**
(45) Date de publication et mention de la délivrance du brevet: **23.12.1998**
(21) Numéro de dépôt: 92921587.9
(22) Date de dépôt: 29.09.1992
(51) Int. Cl.: C07K 14/22, A61K 39/095

(54) **Vaccin comprenant une sous-unité du récepteur de la transferrine humaine tel qu'isolé de Neisseria meningitidis**
Impfstoff, der eine Untereinheit des menschlichen Transferrin-Rezeptors von Neisseria meningitidis enthält
Vaccine comprising a sub-unit of the human transferrrin receptor from Neisseria meningitidis

(30) Priorité: 03.10.1991 FR 9112176
(43) Date de publication de la demande: 22.09.1993
(73) Titulaire: PASTEUR MERIEUX SERUMS ET VACCINS, Société Anonyme :, F-69007 Lyon (FR)
(72) Inventeur: QUENTIN-MILLET, Marie-José, F-69100 Villeurbanne (FR); LISSOLO, Ling, F-69280 Marcy-l'Etoile (FR)
(74) Mandataire: Bernasconi, Jean
(86) Numéro de dépôt international: FR9200904
(87) Numéro de publication internationale: WO9307172

(56) Documents cités:
- WO-A-90/12591
- WO-A-92/03467
- INFECTION AND IMMUNITY vol. 58, no. 9, Septembre 1990, WASHINGTON US pages 2875 - 2881 NIRUPAMA BANERJEE-BHATNAGAR ET AL 'Expression of neisseria meningitidis Iron-regulated outer membrane proteins, including a 70-kilodalton transferrin receptor, and their potential use as vaccines'
- INFECTION AND IMMUNITY vol. 56, no. 5, Mai 1988, WASHINGTON US pages 1144 - 1149 SCHRYVERS A. ET AL 'Identification and characterization of the human lactoferrin-binding protein from neisseria meningitidis'
- CHEMICAL ABSTRACTS, vol. 111, no. 17, 23 Octobre 1989, Columbus, Ohio, US; abstract no. 150244, SCHRYVERS A. ET AL 'Identification and characterization of the transferrin receptor from Neisseria meningitidis'
- FEBS Microbiology Letters, 110, 1993, 51-8

## Description

La présente invention a pour objet une composition pharmaceutique vaccinale destinée à la prévention des méningites causées par *Neisseria meningitidis.*

D'une manière générale, les méningites sont soit d'origine virale, soit d'origine bactérienne. Les bactéries principalement responsables sont : *N. meningitidis* et *Haemophilus influenzae,* respectivement impliquées dans environ 40 et 50 % des cas de méningites bactériennes.

On dénombre en France, environ 600 à 800 cas par an de méningites à *N. meningitidis.* Aux Etats-Unis, le nombre de cas s'élève à environ 2 500 à 3 000 par an.

L'espèce *N*. *meningitidis* est sub-divisée en sérogroupes selon la nature des polysaccharides capsulaires. Bien qu'il existe une douzaine de sérogroupes, 90 % des cas de méningites sont attribuables à 3 sérogroupes : A, B et C.

Il existe des vaccins efficaces à base de polysaccharides capsulaires pour prévenir les méningites à *N*. *meningitidis* sérogroupes A et C. Ces polysaccharides tels quels ne sont que peu ou pas immunogéniques chez les enfants de moins de 2 ans et n'induisent pas de mémoire immunitaire. Toutefois, ces inconvénients peuvent être surmontés en conjuguant ces polysaccharides à une protéine porteuse.

Par contre, le polysaccharide de *N. meningitidis* groupe B n'est pas ou peu immunogène chez l'homme, qu'il soit sous forme conjuguée ou non. Ainsi, il apparait hautement souhaitable de rechercher un vaccin à l'encontre des méningites induites par *N*. *meningitidis* notamment du sérogroupe B autre qu'un vaccin à base de polysaccharide.

A cette fin, différentes protéines de la membrane externe de *N. meningitidis* ont déjà été proposées. Il s'agit en particulier du récepteur membranaire de la transferrine humaine.

D'une manière générale, la grande majorité des bactéries ont besoin de fer pour leur croissance et elles ont développé des systèmes spécifiques d'acquisition de ce métal. En ce qui concerne notamment *N*. *meningitidis* qui est un pathogène strict de l'homme, le fer ne peut être prélevé qu'à partir des protéines humaines de transport du fer telles que la transferrine et la lactoferrine puisque la quantité de fer sous forme libre est négligable chez l'homme (de l'ordre de : 10⁻¹⁸ M), en tout cas insuffisante pour permettre la croissance bactérienne.

Ainsi, *N*. *meningitidis* possède un récepteur de la transferrine humaine et un récepteur de la lactoferrine humaine qui lui permettent de fixer ces protéines chélatrices du fer et de capter par la suite le fer nécessaire à sa croissance.

Le récepteur de la transferrine de la souche *N*. *meningitidis* B16B6 a été purifié par Schryvers et al (WO 90/12591) à partir d'un extrait membranaire. Cette protéine telle que purifiée apparaît essentiellement constituée de 2 types de polypeptides : un polypeptide d'un poids moléculaire apparent élevé de 100 kD et un polypeptide d'un poids moléculaire apparent moindre d'environ 70 kD, tels que révélés après électrophorèse sur gel de polyacrylamide en présence de SDS.

Avant les travaux de Schryvers et al mettant en évidence la constitution exacte du récepteur de la transferrine chez *N. meningitidis*, divers auteurs y compris Schryvers lui-même, ont longtemps pensé que la fonction de récepteur de la transferrine était associée à la protéine majeure de la membrane externe dont le poids moléculaire est en fait très proche de celui de la sous-unité de poids moléculaire moindre du récepteur de la transferrine : 70-71 kD. On cite par exemple, Schryvers & Morris, Mol. Microbiol. (1988) 2 (2) : 281 et BanerjeeBhatnagar et al, Infect. Immun. (1990) 58 (9) : 2875. En utilisant la technique d'électrophorèse sur gel de SDS-PAGE, chacun de ces deux documents met en évidence les nombreuses protéines contenues dans un extrait de membrane externe de *N. meningitidis* parmi lesquelles se trouvent la protéine majeure de 70-71 kD ainsi que les sous-unités du récepteur transferrine (sans qu'elles soient nommées). Par électroblot, les auteurs montrent que l'activité de ligand pour la transferrine est associée avec la bande migrant à 70-71 kD que l'on sait maintenant contenir à la fois la protéine majeure de 70-71 kD et Tbp2. Aucune protéine de membrane externe n'est purifiée au cours des expériences reportées dans Schryvers & Morris et Banerjee-Bhatnagar et al.

Le produit de la purification notamment mise en oeuvre par Schryvers est appelé, par définition arbitraire et pour les besoins de la présente demande de brevet, récepteur de la transferrine et les polypeptides le constituant, des sous-unités. Dans la suite du texte, les sous-unités de poids moléculaire élevé et de poids moléculaire moindre sont respectivement appelées Tbp1 et Tbp2.

De manière surprenante, on a maintenant trouvé que la sous-unité de haut poids moléculaire ne pourrait pas induire la production d'anticorps de type neutralisant. Seule la plus petite des 2 sous-unités du récepteur serait capable de remplir cette fonction.

En conséquence, l'invention propose :
i) La sous-unité de moindre poids moléculaire du récepteur de la transferrine humaine d'une souche de *N. meningitidis,* un fragment ou un analogue de ladite sous-unité, sous forme purifiée ; c'est-à-dire dissociée et isolée de la sous-unité de haut poids moléculaire dudit récepteur ; et
ii) Une composition pharmaceutique vaccinale qui comprend à titre d'agent thérapeutique la sous-unité de moindre poids moléculaire du récepteur de la transferrine humaine d'au moins une souche de *N. meningitidis,* un fragment ou un analogue de ladite sous-unité ; en l'absence de la sous-unité de haut poids moléculaire dudit récepteur ;
et permet l'usage thérapeutique de la sous-unité de moindre poids moléculaire du récepteur de la transferrine humaine d'au moins une souche de *N*. *meningitidis,* un fragment ou un analogue de ladite sous-unité ; en l'absence de la sous-unité de haut poids moléculaire dudit récepteur.

D'une manière générale, la sous-unité de moindre poids moléculaire peut être obtenue sous forme purifiée (c'est-à-dire dissociée et isolée de la sous-unité de haut poids moléculaire) notamment à partir d'un récepteur de la transferrine. Ce dernier peut être isolé à partir d'une souche de *N. meningitidis* préalablement cultivée dans un milieu carencé en fer sous forme libre notamment selon la méthode de Schryvers et al, WO 90/12591, décrite de manière similaire dans Schryvers et al, Infect. Immun. (1988) 56 (5) : 1144. Puis le récepteur purifié est soumis à l'action d'un agent fortement dénaturant tel que l'urée 8M ou la guanidine HCl 6M. Les sous-unités dissociées sont finalement séparées par des méthodes chromatographiques classiques telles qu'une chromatographie d'échange d'ions, une chromatographie hydrophobe ou de gel de filtration.

De manière alternative, la sous-unité de moindre poids moléculaire peut être produite en mettant en oeuvre les techniques du génie génétique. Le fragment d'ADN codant pour cette sous-unité peut être exprimé dans un système d'expression hétérologue (e.g. bactérie, levure, cellule de mammifère). La sous-unité est dans ce cas-là recueillie à partir d'une culture et purifiée. Ces méthodes sont en outre parfaitement adaptées à la production des fragments ou des analogues de la sous-unité.

Par "fragment de la sous-unité de moindre poids moléculaire", on signifie un peptide ayant une séquence d'acides aminés qui est incluse dans la séquence de la sous-unité. Par "analogue de la sous-unité de moindre poids moléculaire", on signifie une protéine ayant une séquence d'acides aminés qui présente un degré d'homologie d'au moins 80 %, de préférence d'au moins 90 %, de manière tout à fait préférée d'au moins 95 % avec la séquence de la sous-unité. Aux fins de la présente invention, il est bien entendu qu'un tel fragment ou un tel analogue doit conserver les propriétés immunogènes de la sous-unité.

Par rapport à la sous-unité Tbp2, les souches de *N. meningitidis* peuvent se répartir en 2 grands groupes :
- celles dont la sous-unité Tbp2 a un poids moléculaire de 65 à 74 kD environ (souches dites de type 2394) ; et
- celles dont la sous-unité Tbp2 a un poids moléculaire de 75 à 90 kD environ (souches dites de type 2169).

D'une manière générale, la sous-unité de moindre poids moléculaire utile aux fins de la présente invention peut avoir pour origine une souche de *N*. *meningitidis* de n'importe quel sérogroupe. De manière avantageuse, elle a pour origine une souche de *N. meningitidis* sérogroupe B. Selon un aspect de l'invention tout à fait préféré, elle a pour origine la souche de *N. meningitidis* B16B6, aussi appelée 2394 (B:2a:P1.2:L2.3) ou M982 aussi appelée 2169 (B:9:P1.9:L3.7) qui sont publiquement disponibles auprès de la Collection de l'Institut Pasteur, 25 rue du Dr Roux 75015 Paris sous les numéros d'enregistrement respectifs CIP 7908 et CIP 7917.

A titre d'exemple, la sous-unité Tbp2 des souches 2394 et 2169 est décrite par référence à sa séquence d'acides aminés telle que montrée dans les identificateurs de séquences n°1 et 2 (SEQ ID N°1 et 2). Les poids moléculaires apparents de ces sous-unités sont respectivement 68-70 et 87 kD environ, tels que révelés après electrophorèse sur gel de polyacrylamide en présence de SDS.

Une composition pharmaceutique selon l'invention est notamment utile pour prévenir ou atténuer les effets d'une infection à *N*. *meningitidis.*

Une composition pharmaceutique selon l'invention peut être fabriquée de manière conventionnelle. En particulier on associe l'agent thérapeutique selon l'invention avec un diluant ou un support acceptable d'un point de vue pharmaceutique. Une composition selon l'invention peut être administrée par n'importe quelle voie conventionnelle en usage dans le domaine des vaccins, en particulier par voie sous-cutanée, par voie intra-musculaire ou par voie intraveineuse, par exemple sous forme de suspension injectable. L'administration peut avoir lieu en dose unique ou répétée une ou plusieurs fois après un certain délai d'intervalle. Le dosage approprié varie en fonction de divers paramètres, par exemple, de l'individu traité ou du mode d'administration.

Enfin une composition selon l'invention peut contenir une ou plusieurs sous-unités de moindre poids moléculaire selon qu'elles proviennent de différentes souches de *N. meningitidis.* Ainsi, selon un aspect particulier de l'invention, une composition pharmaceutique avantageuse comprend la sous-unité de moindre poids moléculaire du récepteur de la transferrine humaine d'une souche de type 2394 (poids moléculaire de 65 à 74 kD) et la sous-unité de moindre poids moléculaire du récepteur de la transferrine humaine d'une souche de type 2169 (poids moléculaire de 75 à 90 kD).

De manière préférée, une composition selon l'invention comprend la sous-unité de moindre poids moléculaire du récepteur de la transferrine humaine de la souche 2394 (poids moléculaire : 68-70 kD) et la sous-unité de moindre poids moléculaire du récepteur de la transferrine humaine de la souche 2169 (poids moléculaire : 87 kD).

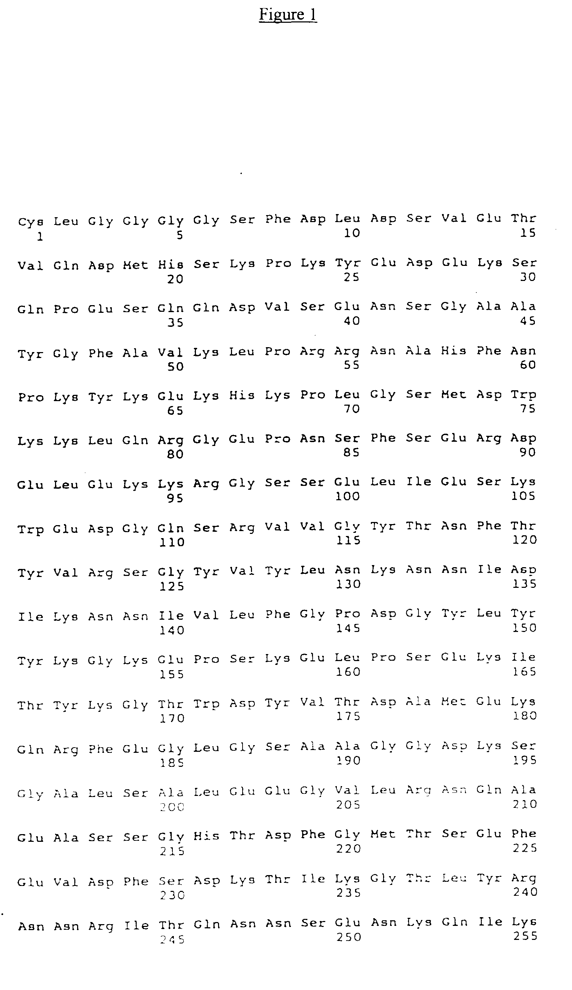

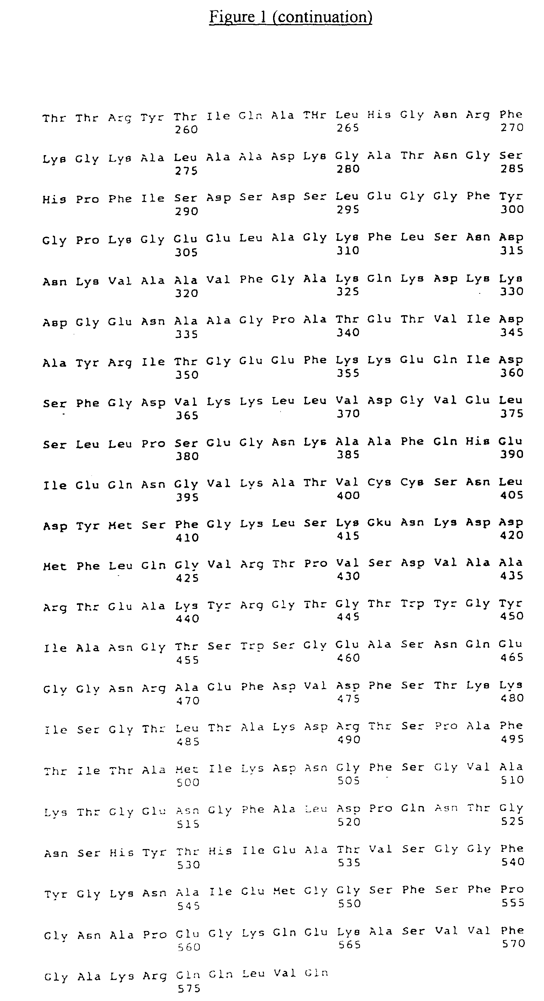

La Figure 1 présente le SEQ ID NO : 1 relatif à la séquence d'acides aminés de la sous-unité Tbp2 de *N. meningitidis* 2394.

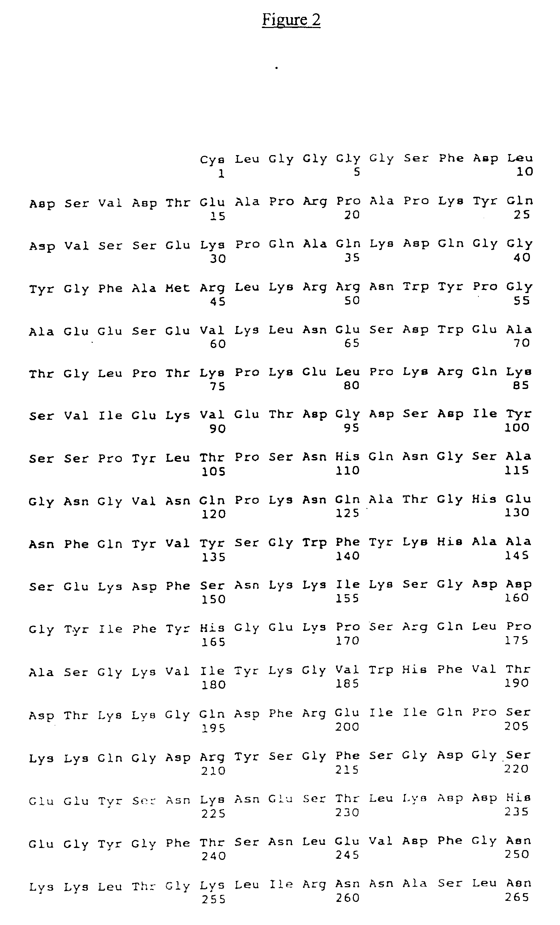

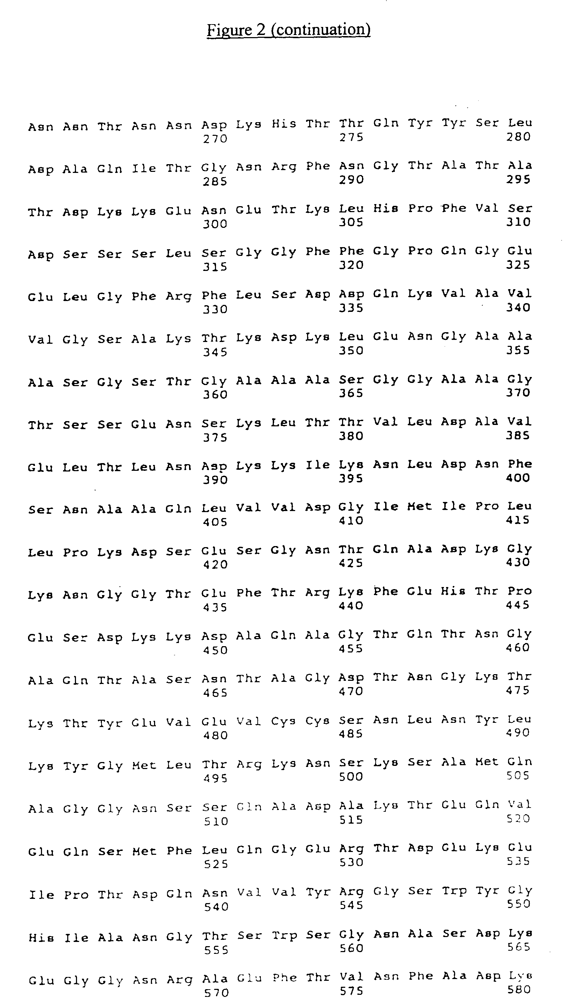

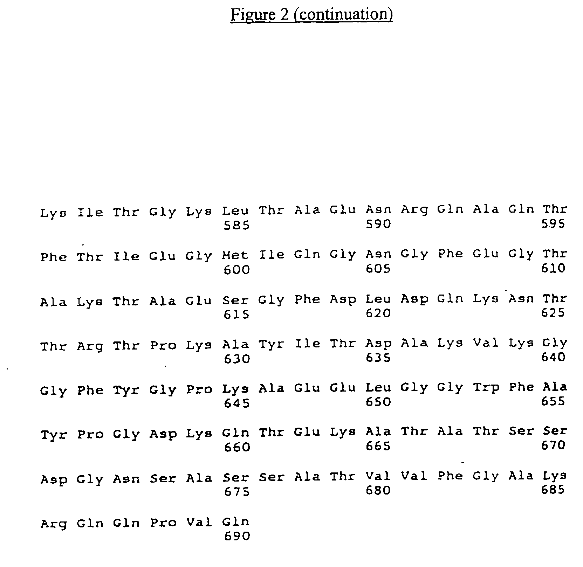

La Figure 2 présente le SEQ ID NO : 2 relatif à la séquence d'acides aminés de la sous-unité Tbp2 de *N. meningitidis* 2169. L'invention est décrite en détails dans les exemples ci-après.

### EXEMPLE 1: Purification de la sous-unité de moindre poids moléculaire du récepteur de la transferrine à partir de la souche 2394, par chromatographie d'échanges d'ions.

### 1A- Culture

Un lyophilisat de la souche *N. meningitidis* 2394 est repris dans environ 1 ml de bouillon Mueller-Hinton (BMH, Difco). La suspension bactérienne est ensuite étalée sur le milieu solide Mueller-Hinton contenant du sang cuit (5 %).

Après 24 h d'incubation à 37°C dans une atmosphère contenant 10 % de CO₂, la nappe bactérienne est recueillie pour ensemencer 150 ml de BMH pH 7,2, répartis en 3 erlens de 250 ml. L'incubation est poursuivie pendant 3 h à 37°C sous agitation. Chacune des 3 cultures ainsi réalisées permet d'ensemencer 400 ml de BMH pH 7,2 supplémentés avec 30 µm d'ethylenediamine-di (o - hydroxyphenylacetic acid) (EDDA, Sigma), qui est un agent chélatant du fer sous forme libre.

Après 16 h de culture à 37°C sous agitation, les cultures sont contrôlées pour leur pureté par observation au microscope après une coloration de Gram. La suspension est centrifugée, le culot contenant les germes est pesé et conservé à - 20°C.

### 1B - Purification

La méthode de purification est essentiellement telle que décrite par Schryvers et al (supra).

Le culot bactérien obtenu en lA est décongelé, puis remis en suspension dans 200 ml de tampon Tris HCI 50 mM, pH 8,0 (tampon A). La suspension est centrifugée pendant 20 min à 15 000 xg à 4°C. Le culot est récupéré, puis remis en suspension dans du tampon A à la concentration finale de 150 g/l. Des fractions de 150 ml sont traitées pendant 8 min à 800 bars dans un lyseur de cellules travaillant sous haute pression (Rannie, modèle 8.30H). Le lysat cellulaire ainsi obtenu est centrifugé pendant 15 min à 4°C à 15 000 xg. Le surnageant est récupéré, puis centrifugé pendant 75 min à 4°C à 200 000 xg.

Après élimination du surnageant, le culot est repris dans du tampon A et après dosage de protéines selon Lowry, la concentration de la suspension est ajustée à 5 mg/ml.

A 1,4 ml de la suspension de membranes on ajoute 1,75 mg de transferrine humaine biotinylée selon le procédé décrit par Schryvers. La concentration finale de la fraction membranaire est de 4 mg/ml. Le mélange est incubé 1 heure à 37°C puis centrifugé à 100 000 xg pendant 75 min à 4°C. Le culot de membranes est repris par le tampon A contenant du NaCl 0,1M et incubé pendant 60 min à température ambiante.

Après solubilisation, on ajoute à cette suspension un certain volume de Sarkosyl (N-Lauroylsarcosine, Sigma) à 30 % (p/v) et d'EDTA 500 mM de façon que les concentrations finales en Sarkosyl et EDTA soient de 0,5 % et 5 mM respectivement. Après une incubation de 15 min à 37°C sous agitation, on ajoute 1 ml de résine streptavidine-agarose (Pierce) préalablement lavée en tampon A. La suspension est incubée 15 min à température ambiante puis centrifugée à 1 000 xg pendant 10 min. La résine est ensuite conditionnée dans une colonne et l'éluat direct est éliminé.

La résine est lavée par 3 volumes de colonne de tampon Tris-HCl 50 mM pH 8,0 contenant NaCI 1M, EDTA 10 mM Sarkosyl 0,5 % (tampon B) puis par un volume de colonne de tampon B contenant de la guanidine HCl 750 mM. Le récepteur de la transferrine est ensuite élué par le tampon Tris-HCl 50mM pH 8,0 contenant NaCI 1M, EDTA 10 mM Sarkosyl 0,05 % et de la guanidine HCI 2M. L'éluat est collecté en fraction dont le volume correspond à 1 Vol., dans des tubes contenant 1 Vol. de Tris HCI 50 mM pH 8,0, NaCI 1M. La densité optique à 280 nm de l'éluat est mesurée en sortie de colonne à l'aide d'un détecteur UV.

Les fractions correspondant au pic d'élution sont recueillies, dialysées contre du tampon phosphate 10 mM, pH 8,0 contenant de l'urée 0,5 M, puis concentrées sur une cellule de concentration de type Amicon équipée de membrane dont le seuil de coupure est de 10 000 Daltons à la concentration finale d'environ 3 mg de protéine/ml.

Une certaine quantité d'urée est ajoutée à la solution concentrée de façon que la concentration finale en urée soit de 8M, la concentration finale de la solution protéique restant comprise entre 2 à 3 mg/ml. La solution est incubée pendant 6 jours à 4°C.

Le mélange est ensuite chromatographié sur une résine échangeuse d'anions (Q-Sépharose Pharmacia) préalablement équilibrée dans le tampon Tris-HCl 50 mM pH 8,0 contenant de l'urée 5M.

Dans ces conditions, la sous-unité de haut-poids moléculaire (Tbpl) est directement recueillie dans l'éluat direct tandis que la sous-unité de poids moléculaire moindre (Tbp2) est éluée par un gradient linéaire de 0 - 1 M NaCI dans le tampon A contenant du Sarkosyl 0,5 % et l'urée 5M. La densité optique à 280 nm est mesurée en sortie de colonne à l'aide d'un détecteur UV.

Les fractions correspondant au pic d'élution sont recueillies, dialysées contre du tampon phosphate 10 mM, pH 8,0 contenant du Sarkosyl 0,05 % et lyophilisées. Le lyophilisat est repris dans de l'eau à une concentration 10 fois supérieure. La solution est dialysée une seconde fois contre du tampon phosphate 50 mM pH 8,0 contenant du Sarkosyl 0,05 % (tampon C) puis la solution est filtrée sur une membrane de porosité 0,22 µm.

Le contenu en protéines est déterminé et ajusté à 1 mg/ml par addition de tampon C, sous conditions aseptiques. Cette préparation est conservée à - 70°C.

### EXEMPLE 2 : Purification de la sous-unité de moindre poids moléculaire du récepteur de la transferrine, à partir de la souche 2169.

La culture de la souche 2169 et la purification de la sous-unité de moindre poids moléculaire du récepteur de la transferrine sont effectuées dans des conditions identiques à celles décrites dans l'Exemple 1.

### EXEMPLE 3 : Purification de la sous-unité de moindre poids moléculaire du récepteur de la transferrine à partir de la souche N. meningitidis 2394 par chromatographie hydrophobe.

La culture de la souche *N*. *meningitidis* 2394, ainsi que les étapes de purification allant jusqu'à la préparation de la suspension membranaire sont effectuées dans des conditions identiques à celles décrites dans l'exemple 1.

A un volume de la suspension de membranes, on ajoute un volume identique de Tris-HCl 50mM pH 8,0 contenant NaCI 2M, EDTA 20 mM, Sarkosyl 1 % (p/v). Le mélange est incubé 15 min à 37°C sous agitation douce. Puis un volume de cette suspension est mis en contact avec un volume identique de résine Sépharose 4B couplée à la transferrine humaine. Cette résine d'affinité a été couplée en greffant de la transferrine humaine (Sigma, St Louis USA) à du Sépharose 4B-CNBr (Pharmacia) selon les recommandations du fabricant. La densité du ligand et de 5 mg transferrine/ml de résine. Le contact se fait en bain pendant 1 h à température ambiante sous agitation rotative douce. La résine est ensuite conditionnée dans une colonne, l'éluat direct est éliminé.

La résine est lavée par 3 volumes de colonnes de tampon Tris-HCI 50 mM pH 8,0 contenant NaCI 1M, EDTA 10 mM Sarkosyl 0,5 % (tampon B) puis par un volume de colonne de tampon B contenant de la guanidine HCI 750 mM. Le récepteur de la transferrine est ensuite élué par le tampon Tris-HCl 50 mM pH 8,0 NaCI lM EDTA 10mM Sarkosyl 0,05 % et guanidine HCI 2M. La densité optique à 280 nm de l'éluat est mesurée en sortie de colonne à l'aide d'un détecteur UV. Les fractions correspondant au pic d'élution sont réunies et la protéine est précipitée par addition de trois volumes d'éthanol refroidi.

Après une nuit d'incubation à +4°C, la protéine est recueillie par centrifugation pendant une heure à 10.000 x g. Le précipité est repris par un certain volume de tampon phosphate 10 mM pH 7,0 contenant NaCl 0,5 M, guanidine-HCl 5 M (tampon D) de façon à ce que la concentration finale en protéine soit d'environ lmg/ml. La solution est mise en contact avec la résine de phényl-Sépharose (Pharmacia) préalablement équilibrée avec le même tampon. L'incubation se fait en bain sous agitation rotative pendant 2 heures à température ambiante. Le gel est ensuite conditionné dans une colonne.

Dans ces conditions, la sous-unité de haut poids moléculaire (Tbp1) est recueillie dans l'éluat direct, tandis que la sous-unité de moindre poids moléculaire (Tbp2) est fixée sur la résine. La colonne est rincée par trois volumes de tampon D puis par 5 volumes de tampon phosphate 10 mM pH 7,0. Tbp2 est éluée par le tampon phosphate 10mM pH 7,0 contenant 0,5% de Sarkosyl. L'excès de Sarkosyl contenu dans le tampon d'élution de Tbp2 est éliminé par précipitation à l'éthanol, la protéine est ensuite reprise dans le tampon phosphate 50 mM pH 8,0 contenant du Sarkosyl 0,05 % (tampon C).

La solution est ensuite filtrée sur une membrane de porosité 0,22 µm. Le contenu en protéine est déterminé et ajusté à 1 mg/ml par addition de tampon C, sous conditions aseptiques. Cette préparation est conservée à -70°C.

### EXEMPLE 4 : Purification de la sous-unité de moindre poids moléculaire à partir de la souche N. meningitidis 2169 par chromatographie hydrophobe.

La culture de la souche *N. meningitidis* 2169 et la purification de la sous-unité de moindre poids moléculaire du récepteur de la transferrine (Tbp2) sont effectuées dans des conditions identiques à celles décrites dans l'exemple 3.

### EXEMPLE 5 : Mise en évidence de l'importance de la sous-unité de moindre poids moléculaire à titre d'agent vaccinal.

On évalue l'activité bactéricide de sérums spécifiquement dirigés contre la sous-unité de moindre poids moléculaire (Tbp2) du récepteur de la transferrine des souches *N*. *meningitidis* 2394 et 2169.

Pour ce faire, les sous-unités Tbp2 ont été préparées par chromatographie hydrophobe, tel que décrit dans les exemples 3 et 4.

Des lapins néo-zélandais albinos reçoivent par voie sous-cutanée et intramusculaire 50µg de Tbp2 isolée de la souche 2394 ou 2169, en présence d'adjuvant complet de Freund (Difco). 21 et 42 jours après la première injection, les lapins reçoivent à nouveau 50 µg de sous-unité Tbp2 purifiée, mais ces fois-ci en présence d'adjuvant incomplet de Freund. 15 jours après la dernière injection, le sérum des animaux est prélevé, puis décomplementé et filtré sur une membrane de porosité 0,45 µm.

Une gamme de dilution de chacun des antisérums anti-Tbp2 2394 et anti-Tbp2 2169 est préparée en milieu M199 (Gibco). 200 µl de chaque dilution sont déposés dans les puits d'une macroplaque de titrage (8x12in.). Un essai témoin est réalisé avec 200 µl de milieu M199. Dans chacun des puits on ajoute (i) 100 µl d'une culture en phase de croissance exponentielle d'une souche de *N*. *meningitidis,* en milieu Mueller-Hinton contenant à 30 µM EDDA et (ii) 100 µl de complément (sérum de jeune lapin dilué).

Après 30 min d'incubation à 37°C sous agitation douce, on ajoute dans chaque puits, 1ml de milieu Mueller-Hinton contenant 1ml d'agar noble en surfusion. Après solidification du milieu, l'incubation est poursuivie 18-24 hrs à 37°C ; puis le nombre d'unités formant des colonies dans chaque puits est évalué. L'inverse de la dernière dilution d'antisérum en présence de laquelle on observe 50 % de lyse par rapport au témoin, correspond au titre bactéricide.

Les résultats sont présentés dans le tableau ci-dessous :

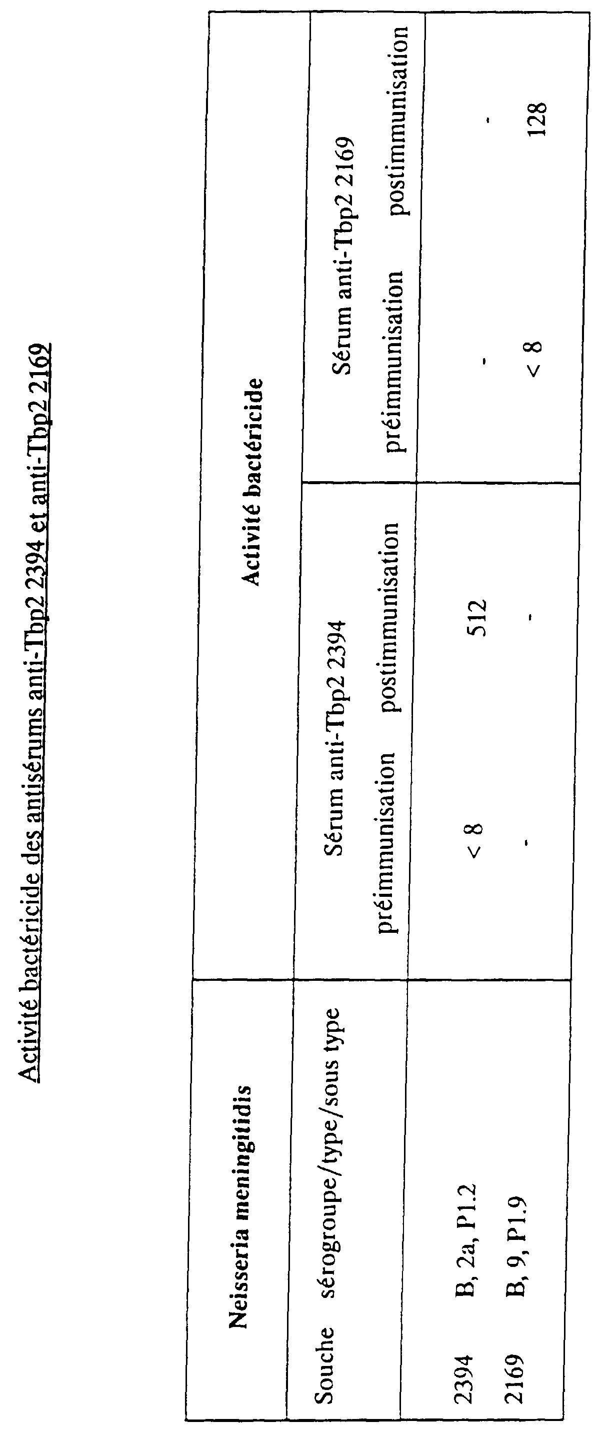

L'antisérum est bactéricide vis-à-vis de la souche à partir de laquelle Tbp2 a été purifiée démontrant que les anticorps anti-Tbp2 induits sont fonctionnels et ont la capacité de lyser la bactérie en présence de complément.

### EXEMPLE 6 : Composition pharmaceutique vaccinale destinée à prévenir des infections à N. meningitidis.

La solution stérile obtenue dans l'Exemple 3 ou 4 est décongelée. Afin de préparer un litre de vaccin renfermant 200 µg/ml d'un principe actif, on mélange stérilement les solutions suivantes :
- Solution contenant la sous-unité Tbp2 du récepteur 2394 (ou 2169) à 1 mg/ml dans du tampon C 200 ml
- Eau physiologique tamponnée (PBS) à pH 6,0 300 ml
- Hydroxyde d'aluminium à 10 mg Al^{+ + +} /ml 50 ml
- Merthiolate à 1 % (p/v) dans du PBS 10 ml
- PBS qsp 1000 ml

### EXEMPLE 7 : Composition pharmaceutique vaccinale destinée à prévenir des infections à N. meningitidis.

Les solutions stériles obtenues dans les Exemples 3 et 4 sont décongelées. Afin de préparer un litre de vaccin renfermant 100 µg/ml de chacun des principes actifs, on mélange stérilement les solutions suivantes :
- Solution contenant la sous-unité Tbp2 du récepteur 2394 à 1mg/ml dans du tampon C 100 ml
- Solution contenant la sous-unité Tbp2 du récepteur 2169 à 1mg/ml dans du tampon C 100 ml
- Eau physiologique tamponnée (PBS) à pH 6,0 300 ml
- Hydroxyde d'aluminium à 10 mg Al^{+ + +} /ml 50 ml
- Merthiolate à 1 % (p/v) dans du PBS 10 ml
- PBS qsp 1000 ml

## Revendications

1. La sous-unité de moindre poids moléculaire du récepteur de la transferrine humaine d'une souche de *N. meningitidis,* sous forme purifiée ; un peptide dont la séquence d'acides aminés dérive par fragmentation de celle de ladite sous-unité ; ou un analogue de ladite sous-unité dont la séquence d'acides aminés présente au moins 80 % d'homologie avec celle de ladite sous-unité, ledit peptide ou analogue ayant conservé les propriétés immunogènes de la sous-unité.

2. La sous-unité de moindre poids moléculaire du récepteur de la transferrine humaine d'une souche de *N. meningitidis* sérogroupe B, sous forme purifiée.

3. La sous-unité de moindre poids moléculaire du récepteur de la transferrine humaine d'une souche de *N*. *meningitidis,* sous forme purifiée ; ladite sous-unité ayant un poids moléculaire de 65 à 74 kD environ.

4. La sous-unité de moindre poids moléculaire du récepteur de la transferrine humaine de la souche de *N*. *meningitidis* 2394 dont la séquence d'acides aminés est telle que montrée dans le SEQ ID NO : 1, sous forme purifiée.

5. La sous-unité de moindre poids moléculaire du récepteur de la transferrine humaine d'une souche de *N*. *meningitidis,* sous forme purifiée; ladite sous-unité ayant un poids moléculaire de 75 à 90 kD environ.

6. La sous-unité de moindre poids moléculaire du récepteur de la transferrine humaine d'une souche de *N. meningitidis* 2169 dont la séquence d'acides aminés est telle que montrée dans le SEQ ID NO : 2, sous forme purifiée.

7. Une composition pharmaceutique vaccinale qui comprend à titre d'agent thérapeutique la sous-unité de moindre poids moléculaire du récepteur de la transferrine humaine d'au moins une souche de *N*. *meningitidis,* un peptide dont la séquence d'acides aminés dérive par fragmentation de celle de ladite sous-unité ; ou un analogue de ladite sous-unité dont la séquence d'acides aminés présente au moins 80 % d'homologie avec celle de ladite sous-unité ; en l'absence de la sous-unité de haut poids moléculaire dudit récepteur.

8. Une composition pharmaceutique vaccinale selon la revendication 7, qui comprend à titre d'agent thérapeutique la sous-unité de moindre poids moléculaire du récepteur de la transferrine humaine d'au moins une souche de *N*. *meningitidis.*

9. Une composition pharmaceutique selon la revendication 8, qui comprend la sous-unité de moindre poids moléculaire du récepteur de la transferrine humaine d'au moins une souche de *N*. *meningitidis* sérogroupe B.

10. Une composition pharmaceutique selon la revendication 8, qui comprend à titre d'agent thérapeutique la sous-unité de moindre poids moléculaire du récepteur de la transferrine humaine d'une souche de *N*. *meningitidis;* ladite sous-unité ayant un poids moléculaire de 65 à 74 kD environ.

11. Une composition pharmaceutique selon la revendication 10, qui comprend à titre d'agent thérapeutique la sous-unité de moindre poids moléculaire du récepteur de la transferrine humaine de *N*. *meningitidis* 2394 dont la séquence d'acides aminés est telle que montrée dans le SEQ ID NO : 1.

12. Une composition pharmaceutique selon la revendication 8, qui comprend à titre d'agent thérapeutique la sous-unité de moindre poids moléculaire du récepteur de la transferrine humaine d'une souche de *N. meningitidis;* ladite sous-unité ayant un poids moléculaire de 75 à 90 kD environ.

13. Une composition pharmaceutique selon la revendication 12, qui comprend à titre d'agent thérapeutique la sous-unité de moindre poids moléculaire du récepteur de la transferrine humaine de *N. meningitidis* 2169 dont la séquence d'acides aminés est telle que montrée dans le SEQ ID NO : 2.

14. Une composition pharmaceutique, qui comprend à titre d'agent thérapeutique :
i) une première sous-unité de moindre poids moléculaire du récepteur de la transferrine humaine d'une première souche de *N. meningitidis;* ladite première sous-unité ayant un poids moléculaire de 65 à 74 kD environ ; et
ii) une seconde sous-unité de moindre poids moléculaire du récepteur de la transferrine humaine d'une seconde souche de *N. meningitidis* ; ladite seconde sous-unité ayant un poids moléculaire de 75 à 90 kD environ ;
en l'absence de la sous-unité de haut poids moléculaire dudit récepteur desdites première et deuxième souches de *N. meningitidis.*

15. Une composition pharmaceutique selon la revendication 14, qui comprend à titre d'agent thérapeutique :
i) la sous-unité de moindre poids moléculaire du récepteur de la transferrine humaine de *N*. *meningitidis* 2394 dont la séquence d'acides aminés est telle que montrée dans le SEQ ID NO : 1 ; et
ii) la sous-unité de moindre poids moléculaire du récepteur de la transferrine humaine de *N. meningitidis* 2169 dont la séquence d'acides aminés est telle que montrée dans le SEQ ID NO : 2 ;
en l'absence de la sous-unité de haut poids moléculaire dudit récepteur des souches de *N. meningitidis* 2394 et 2169.

## Patentansprüche

1. Untereinheit des menschlichen Transferrin-Rezeptors mit dem geringeren Molekulargewicht aus einem N. meningitidis-Stamm in gereinigter Form; Peptid, dessen Aminosäuresequenz von einem Fragment der Sequenz der Untereinheit stammt; oder ein Analogon der Untereinheit, dessen Aminosäuresequenz mindestens 80% Homologie mit der Sequenz der Untereinheit aufweist, wobei die immunogenen Eigenschaften der Untereinheit bei dem Peptid oder Analogon konserviert sind.

2. Untereinheit des menschlichen Transferrin-Rezeptors mit dem geringeren Molekulargewicht aus einem N. meningitidis-Stamm der Serogruppe B in gereinigter Form.

3. Untereinheit des menschlichen Transferrin-Rezeptors mit dem geringeren Molekulargewicht aus einem N. meningitidis-Stamm in gereinigter Form, wobei die Untereinheit ein Molekulargewicht von etwa 65 bis 74 kD hat.

4. Untereinheit des menschlichen Transferrin-Rezeptors mit dem geringeren Molekulargewicht aus dem N. meningitidis-Stamm 2394, deren Aminosäuresequenz die in der SEQ ID NR:1 gezeigte ist, in gereinigter Form.

5. Untereinheit des menschlichen Transferrin-Rezeptors mit dem geringeren Molekulargewicht aus einem N. meningitidis-Stamm in gereinigter Form, wobei die Untereinheit ein Molekulargewicht von etwa 75 bis 90 kD hat.

6. Untereinheit des menschlichen Transferrin-Rezeptors mit dem geringeren Molekulargewicht aus dem N. meningitidis-Stamm 2169, deren Aminosäuresequenz die in der SEQ ID NR:2 gezeigte ist, in gereinigter Form.

7. Pharmazeutische Impfstoffzusammensetzung, umfassend als therapeutisches Mittel die Untereinheit des menschlichen Transferrin-Rezeptors mit dem geringeren Molekulargewicht aus mindestens einem N. meningitidis-Stamm; ein Peptid, dessen Aminosäuresequenz von einem Fragment der Sequenz der Untereinheit stammt; oder ein Analogon der Untereinheit, dessen Aminosäuresequenz mindestens 80% Homologie mit derjenigen der Untereinheit aufweist, in Abwesenheit der Untereinheit mit hohem Molekulargewicht des Rezeptors.

8. Pharmazeutische Impfstoffzusammensetzung nach Anspruch 7, umfassend als therapeutisches Mittel die Untereinheit des menschlichen Transferrin-Rezeptors mit dem geringeren Molekulargewicht aus mindestens einem N. meningitidis-Stamm.

9. Pharmazeutische Zusammensetzung nach Anspruch 8, umfassend die Untereinheit des menschlichen Transferrin-Rezeptors mit dem geringeren Molekulargewicht aus mindestens einem N. meningitidis-Stamm der Serogruppe B.

10. Pharmazeutische Zusammensetzung nach Anspruch 8, umfassend als therapeutisches Mittel die Untereinheit des menschlichen Transferrin-Rezeptors mit dem geringeren Molekulargewicht aus einem N. meningitidis-Stamm, wobei die Untereinheit ein Molekulargewicht von etwa 65 bis 74 kD hat.

11. Pharmazeutische Zusammensetzung nach Anspruch 10, umfassend als therapeutisches Mittel die Untereinheit des menschlichen Transferrin-Rezeptors mit dem geringeren Molekulargewicht aus N. meningitidis 2394, deren Aminosäuresequenz die in der SEQ ID NR:1 gezeigte ist.

12. Pharmazeutische Zusammensetzung nach Anspruch 8, umfassend als therapeutisches Mittel die Untereinheit des menschlichen Transferrin-Rezeptors mit dem geringeren Molekulargewicht aus einem N. meningitidis-Stamm, wobei die Untereinheit ein Molekulargewicht von etwa 75 bis 90 kD hat.

13. Pharmazeutische Zusammensetzung nach Anspruch 12, umfassend als therapeutisches Mittel die Untereinheit des menschlichen Transferrin-Rezeptors mit dem geringeren Molekulargewicht aus N. meningitidis 2169, deren Aminosäuresequenz die in der SEQ ID NR:2 gezeigte ist.

14. Pharmazeutische Zusammensetzung, umfassend als therapeutisches Mittel:
i) eine erste Untereinheit des menschlichen Transferrin-Rezeptors mit dem geringeren Molekulargewicht aus einem ersten N. meningitidis-Stamm, wobei diese erste Untereinheit ein Molekulargewicht von etwa 65 bis 74 kD hat; und
ii) eine zweite Untereinheit des menschlichen Transferrin-Rezeptors mit dem geringeren Molekulargewicht aus einem zweiten N. meningitidis-Stamm, wobei diese zweite Untereinheit ein Molekulargewicht von etwa 75 bis 90 kD hat; in Abwesenheit der Untereinheit mit hohem Molekulargewicht des Rezeptors des ersten und des zweiten N. meningitidis-Stammes.

15. Pharmazeutische Zusammensetzung nach Anspruch 14, umfassend als therapeutisches Mittel:
i) die Untereinheit des menschlichen Transferrin-Rezeptors mit dem geringeren Molekulargewicht aus N. meningitidis 2394, deren Aminosäuresequenz die in der SEQ ID NR:1 gezeigte ist; und
ii) die Untereinheit des menschlichen Transferrin-Rezeptors mit dem geringeren Molekulargewicht aus N. meningitidis 2169, deren Aminosäuresequenz die in der SEQ ID NR:2 gezeigte ist;
in Abwesenheit der Untereinheit mit hohem Molekulargewicht des Rezeptors der Stämme N. meningitidis 2394 und 2169.

## Claims

1. The sub-unit of less molecular weight of the receptor of human transferrin of a branch of N. *Meningitidis,* in a purified form; a peptide whose sequence of amino acids is derived by fragmentation of that of the said sub-unit; or an analog of the said sub-unit whose sequence of amino acids has at lest 80% homology with that of the said sub-unit, the said peptide or analog having retained the immunogenic properties of the sub-unit.

2. The sub-unit of less molecular weight of the receptor of human transferrin of a branch of *N. Meningitidis* serum group B in a purified form.

3. The sub-unit of less molecular weight of the receptor of human transferrin of a branch of *N. Meningitidis,* in a purified form; said sub-unit having a molecular weight of 65 to 74 kD approximately.

4. The sub-unit of less molecular weight of the receptor of human transferrin of a branch of *N. Meningitidis* 2394, whose sequence of amino acids is as shown in SEQ ID NO. 1, in a purified form.

5. The sub-unit of less molecular weight of the receptor of human transferrin of a branch of *N. Meningitidis* in a purified form; the said sub-unit having a molecular weight of 75 to 90 kD approximately.

6. The sub-unit of less molecular weight of the receptor of human transferrin of a branch of *N. Meningitidis* 2169 whose sequence of amino acids is as shown in SEQ ID NO. 2 in a purified form.

7. A vaccinal pharmaceutical compound which comprises as a therapeutic agent the sub-unit of less molecular weight of the receptor of human transferrin of at least one branch of *N. Meningitidis,* a peptide whose sequence of amino acids is derived by fragmentation of that of the said sub-unit; or an analog of the said sub-unit whose sequence of amino acids has at least 80% homology with that of the said sub-unit; in the absence of the sub-unit of high molecular weight of said receptor.

8. A vaccinal pharmaceutical compound according to claim 7, which comprises as a therapeutic agent the sub-unit of less molecular weight of the receptor of human transferrin of at least one branch of *N. Meningitidis.*

9. A pharmaceutical compound according to claim 8, which comprises the sub-unit of less molecular weight of the receptor of human transferrin of at least one branch of *N. Meningitidis* serum group B.

10. A pharmaceutical compound according to claim 8, which comprises as a therapeutic agent the sub-unit of less molecular weight of the receptor of human transferrin of a branch of *N. Meningitidis*, soad sub-unit having a molecular weight of 65 to 74 kD approximately.

11. A pharmaceutical compound according to claim 10, which comprises as a therapeutic agent the sub-unit of less molecular weight of the receptor of human transferrin of *N. Meningitidis* 2394, whose sequence of amino acids is as shown in SEQ ID NO. 1.

12. A pharmaceutical compound according to claim 8, which comprises as a therapeutic agent the sub-unit of less molecular weight of the receptor of human transferrin of a branch of *N. Meningitidis;* said sub-unit having a molecular weight of 75 to 90 kD approximately.

13. A pharmaceutical compound according to claim 12, which comprises as a therapeutic agent the sub-unit of less weight of the receptor of human transferrin of *N. Meningitidis* 2169 whose sequence of amino acids is as shown in SEQ ID NO. 2.

14. A pharmaceutical compound, which comprises as a therapeutic agent:
i) a first sub-unit of less molecular weight of the receptor of human transferrin of a first branch of *N. Meningitidis;* said first sub-unit having a molecular weight of 65 to 74 kD approximately; and
ii) a second sub-unit of less molecular weight of the receptor of human transferrin of a second branch of *N. Meningitidis,* the said second sub-unit having a molecular weight of 75 to 90 kD approximately;
in the absence of the sub-unit of high molecular weight of the said receptor of the said first and second branches of *N. Meningitidis.*

15. A pharmaceutical compound according to claim 14, which comprises as a therapeutic agent:
i) the sub-unit of less molecular weight of the receptor of human transferrin of *N. Meningitidis* 2394 whose sequence of amino acids is as shown in SEQ ID NO 1; and
ii) the sub-unit of less molecular weight of the receptor of human transferrin of *N. Meningitidis* 2169 whose sequence of amino acids is as shown in SEQ ID NO 2;
in the absence of the sub-unit of high molecular weight of said receptor of the branches of *N. Meningitidis* 2394 and 2169.
